# FASCICULE DE BREVET EUROPEEN

(11) **EP 0 898 956 B1**
(45) Date de publication et mention de la délivrance du brevet: **04.10.2001**
(21) Numéro de dépôt: 98402036.2
(22) Date de dépôt: 11.08.1998
(51) Int. Cl.: A61K 7/42, A61K 7/135, A61K 7/48, A61K 7/06

(54) **Utilisation de dérivés oxamates comme agents dépigmentants**
Verwendung von Oxamatderivaten als Depigmentierungsmittel
Use of oxamate compounds as depigmenting agents

(30) Priorité: 26.08.1997 FR 9710657
(43) Date de publication de la demande: 03.03.1999
(73) Titulaire: L'OREAL, 75008 Paris (FR)
(72) Inventeur: Tuloup, Rémy, 75014 Paris (FR); Philippe, Michel, 91320 Wissous (FR)
(74) Mandataire: Tezier Herman, Béatrice

(56) Documents cités:
- EP-A- 0 712 856
- EP-A- 0 722 715
- WO-A-97/20546
- CH-A- 512 257

## Description

La présente invention se rapporte à l'utilisation de dérivés oxamates comme agent dépigmentant ou blanchissant dans une composition cosmétique et/ou dermatologique, ainsi qu'à une composition dépigmentante et/ou blanchissante contenant des dérivés oxamates.

La couleur de la peau humaine est fonction de différents facteurs et notamment des saisons de l'année, de la race et du sexe, et elle est principalement déterminée par la nature et la concentration de mélanine produite par les mélanocytes. Les mélanocytes sont les cellules spécialisées qui par l'intermédiaire d'organelles particuliers, les mélanosomes, synthétisent la mélanine. En outre, à différentes périodes de leur vie, certaines personnes voient apparaître sur la peau et plus spécialement sur les mains, des taches plus foncées et/ou plus colorées, conférant à la peau une hétérogénéité. Ces taches sont dues aussi à une concentration importante de mélanine dans les kératinocytes situés à la surface de la peau.

De la même manière, la couleur des poils et des cheveux est due à la mélanine, lorsque les poils ou les cheveux sont foncés, certaines personnes désirent voir ceux-ci plus claires. Ceci est particulièrement intéressant pour les poils qui sont moins visibles lorsqu'ils sont clairs que lorsqu'ils sont foncés.

Le mécanisme de formation de la pigmentation de la peau, des poils et des cheveux, c'est-à-dire de !a formation de la mélanine est particulièrement complexe et fait intervenir schématiquement les principales étapes suivantes :
Tyrosine ---> Dopa ---> Dopaquinone --> Dopachrome ---> Mélanine

La tyrosinase (monophénol dihydroxyl phénylalanine : oxygen oxydo-reductase EC 1.14.18.1) est l'enzyme essentielle intervenant dans cette suite de réactions. Elle catalyse notamment la réaction de transformation de la tyrosine en Dopa (dihydroxyphénylalanine) grâce à son activité hydroxylase et la réaction de transformation de la Dopa en dopaquinone grâce à son activité oxydase. Cette tyrosinase n'agit que lorsqu'elle est à l'état de maturation sous l'action de certains facteurs biologiques.

Une substance est reconnue comme dépigmentante si elle agit directement sur la vitalité des mélanocytes épidermiques où se déroule la mélanogénèse et/ou si elle interfère avec une des étapes de la biosynthèse de la mélanine soit en inhibant une des enzymes impliquées dans la mélanogénèse soit en s'intercalant comme analogue structural d'un des composés chimiques de la chaîne de synthèse de la mélanine, chaîne qui peut alors être bloquée et ainsi assurer la dépigmentation.

Les substances les plus utilisées en tant que dépigmentants sont plus particulièrement l'hydroquinone et ses dérivés, en particulier ses éthers tels que le monométhyléther et le monoéthyléther d'hydroquinone. Ces composés, bien qu'ils présentent une efficacité certaine, ne sont malheureusement pas exempts d'effets secondaires du fait de leur toxicité, ce qui peut rendre leur emploi délicat, voire dangereux. Cette toxicité provient de ce qu'ils interviennent sur des mécanismes fondamentaux de la mélanogénèse en tuant des cellules qui risquent alors de perturber leur environnement biologique et qui par conséquent obligent la peau à les évacuer en produisant des toxines.

Ainsi, l'hydroquinone est un composé particulièrement irritant et cytotoxique pour le mélanocyte, dont le remplacement, total ou partiel a été envisagé par de nombreux auteurs.

On a ainsi cherché des substances qui n'interviennent pas dans le mécanisme de la mélanogénèse mais qui agissent en amont sur la tyrosinase en empêchant son activation et sont de ce fait beaucoup moins toxiques. On utilise couramment comme inhibiteur de l'activation de la tyrosinase l'acide kojique qui complexe le cuivre présent dans le site actif de cette enzyme. Malheureusement, ce composé peut provoquer des réactions d'allergie ("Contact allergy to kojic acid in skin care products", Nakagawa M. et al., in Contact Dermatitis, Jan. 95, Vol 42 (1), pp.9-13). Ce composé est également instable en solution, ce qui complique quelque peu la fabrication de la composition.

L'utilisation de substances dépigmentantes topiques inoffensives présentant une bonne efficacité est tout particulièrement recherchée en vue de traiter les hyperpigmentations régionales par hyperactivité mélanocytaire telles que les mélasmas idiopathiques, survenant lors de la grossesse ("masque de grossesse" ou chloasma) ou d'une contraception oestro-progestative, les hyperpigmentations localisées par hyperactivité et prolifération mélanocytaire bénigne, telles que les taches pigmentaires séniles dites lentigo actiniques, les hyperpigmentations ou dépigmentations accidentelles, éventuellement dues à la photosensibilisation ou à la cicatrisation post-lésionnelle, ainsi que certaines leucodermies, telles que le vitiligo. Pour ces dernières (les cicatrisations pouvant aboutir à une cicatrice donnant à la peau un aspect plus blanc et les leucodermies), à défaut de pouvoir repigmenter la peau lésée, on achève de dépigmenter les zones de peau normale résiduelle pour donner à l'ensemble de la peau une teinte blanche homogène.

Aussi, il subsiste le besoin d'un nouvel agent blanchissant de la peau humaine, des poils et/ou des cheveux à action aussi efficace que ceux connus, mais n'ayant pas leurs inconvénients, c'est-à-dire qui soit non irritant, non toxique et/ou non allergisant pour la peau et stable dans une composition.

La demanderesse a trouvé de manière inattendue que certains dérivés oxamates présentent une activité dépigmentante, même à faibles concentrations, sans faire preuve de cytotoxicité.

Ces composés sont connus notamment pour leurs propriétés stabilisatrices de polymères (EP 0511166) ou pour des activités biologiques, telles qu' antiinflammatoires (Pharmacie, 35(7), 394-8, 1980), inhibitrices de la neuraminidase (Hoppe-Seyler's Physiol. Chem., 358(3), 391-6, 1977) ou encore cholagogue (Khim-Farm. Zh., 18(6), 683-6, 1984)

Des dérivés d'acide oxamique N-substitués sont également décrits dans EP-A-0 722 715 comme inhibiteurs de la lactate déhydrogénase, pour une utilisation dans le traitement de différents signes du photo-vieillissement par augmentation de la prolifération des cellules de la peau et de la synthèse de collagène.

La présente invention a donc pour objet l'utilisation de dérivés oxamates de formule (I) pour l'obtention d'une composition destinée à dépigmenter et/ou blanchir la peau humaine et/ou enlever les taches pigmentaires de la peau et/ou dépigmenter les poils et/ou les cheveux.

Ces dérivés oxamates présentent la formule (I) suivante : 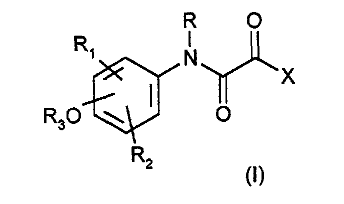 dans laquelle :
R représente un atome d'hydrogène, un radical choisi parmi le radical hydroxyle, un radical -OR', un radical -COR', un radical -COOR', un radical - NR'R", un radical -CONR'R", un radical hydrocarboné aliphatique, saturé ou insaturé, linéaire, cyclique ou ramifié en C₁ à C₃₀, éventuellement hydroxylé, et un radical aryle substitué ou non,
R₁ et R₂, identiques ou différents, représentent un atome d'hydrogène, un radical choisi parmi le radical hydroxyle, un radical -OR', un radical -COR', un radical -COOR', un radical -NR'R", un radical -CONR'R", un radical -SR',-CH₂OR', un radical hydrocarboné aliphatique, saturé ou insaturé, linéaire, cyclique ou ramifié en C₁ à C₃₀, éventuellement hydroxylé, et un radical aryle substitué ou non,
avec R' et R", identiques ou différents, représentant un atome d'hydrogène, un radical choisi parmi un radical hydrocarboné aliphatique, saturé ou insaturé, linéaire ou ramifié en C₁ à C₃₀, éventuellement hydroxylé, et un radical aryle substitué ou non, un résidu d'aminoacide ou de sucre,
R₃ représente un atome d'hydrogène,
X représente un radical choisi parmi un radical -OR₅, un radical SR₅ et un radical -NR₆R₇, avec R₅, R₆ et R₇, identiques ou différents, représentant un atome d'hydrogène, un radical choisi parmi un radical hydrocarboné aliphatique, saturé ou insaturé, linéaire, cyclique ou ramifié en C₁ à C₃₀, éventuellement hydroxylé, et un radical aryle substitué ou non, un résidu d'aminoacide, de peptide ou de sucre.

Ces composés sont donc déjà décrits notamment dans les documents cités ci-dessus. Ils présentent l'avantage d'être faciles à obtenir à partir de précurseurs simples, tels que les halogénures d'oxalyle, l'oxamide ou les esters de l'acide oxamique.

Selon la présente invention, parmi les radicaux hydrocarbonés aliphatiques linéaires ou ramifiés ayant de 1 à 30 atomes de carbone, on peut citer avantageusement les radicaux méthyle, éthyle, propyle, isopropyle, butyle, tertiobutyle, hexyle, octyle, nonyle, 2-éthyl-hexyl et dodécyle. De préférence, ces radicaux présentent de 1 à 12 atomes de carbone. De manière encore plus préférentielle, le radical hydrocarboné aliphatique comprend généralement de 1 à 6 atomes de carbone. On peut citer, comme radical hydrocarboné aliphatique inférieur, les radicaux méthyle, éthyle, propyle, isopropyle, tertiobutyle, hexyle.

Parmi les radicaux hydrocarbonés aliphatiques linéaires ayant de 1 à 30 atomes de carbone, on peut citer notamment les radicaux méthyle, éthyle, propyle, 2-éthyl-hexyle, octyle, dodécyle, hexadécyle et octadécyle.

Parmi les radicaux hydrocarbonés aliphatiques ramifiés ayant de 1 à 30 atomes de carbone, on peut citer notamment les radicaux 2-méthylbutyle, 2-méthylpentyle, 1-méthylhexyle, 3-méthylheptyle.

Lorsqu'il est insaturé, on préfère un radical présentant une ou plusieurs insaturations éthyléniques, tel que plus particulièrement le radical allyle.

Lorsque la radical hydrocarboné aliphatique est cyclique, on peut notamment citer le radical cyclohexyle, cholestéryle ou terbutylcyclohexyle.

Lorsqu'il est hydroxylé, le radical comprend de préférence 1 à 6 atomes de carbone et 1 à 5 groupes hydroxyles.

Parmi les radicaux monohydroxyalkyle, on préfère un radical contenant de préférence 1 ou 3 atomes de carbone, notamment les radicaux hydroxyméthyl, 2-hydroxyéthyl, 2 ou 3-hydroxypropyle.

Parmi les radicaux polyhydroxyalkyle, on préfère un radical présentant de 3 à 6 atomes de carbone et de 2 à 5 groupes hydroxyles, tels que les radicaux 2,3-dihydroxypropyle, 2,3,4-trihydroxybutyle, 2,3,4,5-tétrahydroxypentyle ou le reste du pentaérythritol.

Les radicaux alkoxylés sont des radicaux hydrocarbonés aliphatiques, tels que notamment décrits ci-dessus, précédés d'un atome d'oxygène.

Parmi les radicaux aryle, on préfère un radical phényle, thiophène ou pyridine, éventuellement substitué par au moins un atome d'halogène, un radical hydroxyle, un radical hydrocarboné aliphatique, une fonction nitro, un groupe méthoxy ou une fonction amine éventuellement substituée. On préfère le radical phényle éventuellement substitué.

Par résidu de sucre, on entend un reste dérivant notamment de glucose, de galactose ou de mannose, ou bien encore de l'acide glucuronique.

Par résidu d'aminoacide, on entend notamment un reste dérivant de l'un des acides aminés tels que la lysine, la glycine ou l'acide aspartique, et par reste de peptide, on entend plus particulièrement un reste de dipeptide ou de tripeptide résultant de la combinaison d'acides aminés.

De préférence, les dérivés oxamates utilisés dans la présente invention sont ceux pour lesquels l'une au moins et de préférence toutes les conditions ci-dessous sont respectées :
- R₃ représente un atome d'hydrogène,
- la fonction -OR₃ sur le radical phényl est en position ortho ou avantageusement en position para,
- R est un atome d'hydrogène ou un radical hydrocarboné aliphatique linéaire ou ramifié de C₁-C₂₄, éventuellement insaturé ou hydroxylé,
- X est un radical -OR₅, avec avantageusement R₅ représentant un atome d'hydrogène ou un radical hydrocarboné aliphatique linéaire ou ramifié de C₁-C₂₄, éventuellement insaturé ou hydroxylé, ou X est un radical -NR₆R₇, avec avantageusement R₆ et R₇, identiques ou différents, représentant un atome d'hydrogène, un radical hydrocarboné aliphatique linéaire ou ramifié de C₁-C₂₄, éventuellement insaturé ou hydroxylé, ou un résidu d'aminoacide, de peptide ou de sucre.

La présente invention a aussi pour objet l'utilisation cosmétique de ces dérivés oxamates dans une composition cosmétique, comme inhibiteur de la tyrosinase et/ou de la synthèse de la mélanine.

La présente invention a aussi pour objet l'utilisation d'une composition cosmétique comprenant au moins un de ces dérivés d'oxamate comme composition topique dépigmentante et/ou blanchissante de la peau humaine, des poils ou des cheveux.

La présente invention se rapporte également à un procédé cosmétique et/ou dermatologique de dépigmentation et/ou de blanchiment de la peau humaine, des poils ou des cheveux consistant à appliquer sur la peau, les poils ou les cheveux une composition comprenant au moins un dérivé d'oxamate de formule (1) tel que décrit à-dessus.

La composition selon l'invention est appropriée pour une utilisation topique et contient donc un milieu cosmétiquement ou dermatologiquement acceptable, c'est-à-dire compatible avec la peau, les poils ou les cheveux.

Les dérivés oxamates de formule (I) peuvent être notamment présents dans la composition en une quantité allant de 0,01 à 10 % et de préférence de 0,005 à 5 % du poids total de la composition.

La composition de l'invention peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau ou sur les cheveux sous forme d'aérosol. Elle peut également se présenter sous forme solide, et par exemple sous forme de stick. Elle peut être utilisée comme produit de soin et/ou comme produit de maquillage. Elle peut également être sous une forme de shampooings ou après-shampooings.

De façon connue, la composition de l'invention peut contenir également les adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple les esters d'acide gras et de polyéthylène glycol tels que le stéarate de PEG-20, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, on peut utiliser notamment les polyols (glycérine, propylène glycol), les vitamines, les agents kératolytiques et/ou desquamants (acide salicylique et ses dérivés, alpha-hydroxyacides, acide ascorbique et ses dérivés), les agents anti-inflammatoires, les agents apaisants et leurs mélanges. On peut également associer les dérivés d'oxamate à d'autres agents dépigmentants, tels que l'acide kojique ou l'hydroquinone et ses dérivés, ce qui permet d'utiliser ces derniers à des doses moins toxiques pour la peau. En cas d'incompatibilité, ces actifs et/ou les dérivés d'oxamate peuvent être incorporés dans des sphérules, notamment des vésicules ioniques ou non-ioniques et/ou des nanoparticules (nanocapsules et/ou nanosphères), de manière à les isoler les uns des autres dans la composition.

L'invention va maintenant être illustrée à l'aide des exemples qui suivent. Les concentrations sont données en pourcentage en poids.

### Exemples de composés

### Exemple 1

### N-(4-hydroxyphényl)oxamate d'éthyle

250g de paraaminophénol dans un litre d'oxalate d'éthyle est porté à 120°C pendant 4 heures. Après, refroidissement, le mélange réactionnel est additionné d'un litre d'éthanol absolu et l'ensemble est refroidi à 0°C. Le N-(4-hydroxyphényl)oxamate d'éthyle précipite, il est filtré et lavé par de l'heptane, puis séché sous vide. Le rendement est de 91%.

### Exemple 2

### N-(4-hydroxyphényl)-N'-(D-glucamino)oxamide

2g de D-glucamine et 2g de N-(4-hydroxyphényl)oxamate d'éthyle sont portés au reflux de 50 cm3 d'éthanol pendant 3 heures. Le milieu est ensuite refroidi à 0°C. L'oxamide attendu précipite avec un rendement de 50%.

### Tests :

Un test biologique a mis en évidence l'activité dépigmentante des dérivés d'oxamate de formule (I)

Ce test correspond à celui décrit dans le brevet FR 2734825 déposé par la Demanderesse, ainsi que dans l'article de R. Schmidt, P. Krien et M. Régnier, Anal. Biochem., 235(2), 113-18, (1996). Ce test est ainsi réalisé sur coculture de kératinocytes et de mélanocytes.

Pour chaque composé testé, il est déterminé la valeur de IC50 qui correspond à la concentartion micromolaire (µM) pour laquelle est observée 50% d'inhibition de la mélanogénèse.

Par ailleurs, une classe est donnée à chacun de ces composés pour leur activité dépigmentante maximale :
classe 1 : 10 à 30% d'inhibition de la mélanogénèse par rapport au témoin (même expérience sans composé à tester) ;
classe 2 : 30 à 60% d'inhibition de la mélanogénèse par rapport au témoin (même expérience sans composé à tester) ;
classe 3 : 60 à 100% d'inhibition de la mélanogénèse par rapport au témoin (même expérience sans composé à tester).

Les résultats sont rassemblés dans le tableau (1) suivant.

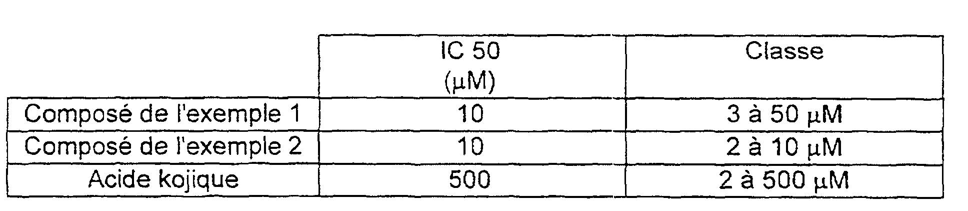

Ces composés de formule (1) présentent donc une plus grande efficacité dépigmentante que l'acide kojique. En outre, ils ont l'avantage de ne pas présenter de cytotoxicité à l'égard des kératinocytes et les mélanocytes, défaut majeur des dépigmentants existants.

### Exemples de compositions

### Exemple 3 : Crème traitante

- Alcool cétylique 1,05 %
- Stéarate de PEG-20 (Myrj 49 vendu par la société ICI) 2 %
- Cyclométhicone 6 %
- Composé de l'exemple 1 0,5 %
- Carbomer 0,6 %
- Glycérine 3 %
- Triéthanolamine 1 %
- Conservateurs 0,5 %
- Eau déminéralisée qsp 100 %

La crème obtenue utilisée en application quotidienne, permet d'obtenir un blanchiment de la peau.

### Exemple 2 : Gel traitant

- Propylène glycol 10 %
- Alcool éthylique 40 %
- Glycérine 3 %
- Composé de l'exemple 2 0,5 %
- Conservateurs 0,15 %
- Parfum 0,15 %
- Eau déminéralisée qsp 100 %

Le gel obtenu peut être utilisé quotidiennement et est apte à dépigmenter la peau.

### Exemple 3 : Stick traitant

- Cire de Carnauba 5 %
- Ozokerite 7 %
- Lanoline 6 %
- Dioxyde de titane (pigments) 20 %
- Amidon de riz (charge) 7 %
- EDTA 0,1 %
- Composé de l'exemple 1 2 %
- Perhydrosqualène qsp 100 %

Le stick obtenu, utilisé sur les taches pigmentaires, permet de les atténuer voire de les faire disparaître.

## Revendications

1. Utilisation d'au moins un dérivé d'oxamate présentant la formule (I) suivante 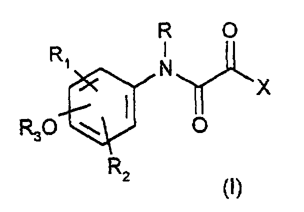 dans laquelle
R représente un atome d'hydrogène, un radical choisi parmi le radical hydroxyle, un radical -OR', un radical -COR', un radical -COOR', un radical-NR'R", un radical -CONR'R", un radical hydrocarboné aliphatique, saturé ou insaturé, linéaire, cyclique ou ramifié en C₁ à C₃₀, éventuellement hydroxylé, et un radical aryle substitué ou non,
R₁ et R₂, identiques ou différents, représentent un atome d'hydrogène, un radical choisi parmi le radical hydroxyle, un radical -OR', un radical -COR', un radical -COOR', un radical -NR'R", un radical -CONR'R", un radical -SR',-CH₂OR', un radical hydrocarboné aliphatique, saturé ou insaturé, linéaire, cyclique ou ramifié en C₁ à C₃₀, éventuellement hydroxylé, et un radical aryle substitué ou non, avec R' et R", identiques ou différents, représentant un atome d'hydrogène, un radical choisi parmi un radical hydrocarboné aliphatique, saturé ou insaturé, linéaire ou ramifié en C₁ à C₃₀, éventuellement hydroxylé, et un radical aryle substitué ou non, un résidu d'aminoacide ou de sucre,
R₃ représente un atome d'hydrogène,
X représente un radical choisi parmi un radical -OR₅, un radical SR₅ et un radical -NR₆R₇,
avec R₅, R₆ et R₇, identiques ou différents, représentant un atome d'hydrogène, un radical choisi parmi un radical hydrocarboné aliphatique, saturé ou insaturé, linéaire, cyclique ou ramifié en C₁ à C₃₀, éventuellement hydroxylé, et un radical aryle substitué ou non, un résidu d'aminoacide, de peptide ou de sucre, pour l'obtention d'une composition destinée à dépigmenter et/ou blanchir la peau humaine et/ou enlever les taches pigmentaires de la peau et/ou dépigmenter les poils et/ou les cheveux.

2. Utilisation cosmétique d'au moins un dérivé d'oxamate présentant la formule (I), telle que définie dans la revendication 1, dans une composition cosmétique, comme inhibiteur de la tyrosinase et/ou de la synthèse de la mélanine.

3. Utilisation d'une composition cosmétique comprenant au moins un dérivé d'oxamate présentant la formule (I), telle que définie dans la revendication 1, comme composition topique dépigmentante et/ou blanchissante de la peau humaine, des poils ou des cheveux.

4. Utilisation selon l'une quelconque des revendications précédentes, **caractérisée en ce que** les dérivés d'oxamate sont choisis parmi ceux pour lesquels l'une au moins et de préférence toutes les conditions ci-dessous sont respectées :
- R₃ représente un atome d'hydrogène,
- la fonction -OR₃ sur le radical phényl est en position ortho ou avantageusement en position para,
- R est un atome d'hydrogène ou un radical hydrocarboné aliphatique linéaire ou ramifié de C₁-C₃₀, éventuellement insaturé ou hydroxylé,
- X est un radical -OR₅, avec avantageusement R₅ représentant un atome d'hydrogène ou un radical hydrocarboné aliphatique linéaire ou ramifié de C₁-C₃₀, éventuellement insaturé ou hydroxylé, ou X est un radical -NR₆R₇, avec avantageusement R₆ et R₇, identiques ou différents, représentant un atome d'hydrogène, un radical hydrocarboné aliphatique linéaire ou ramifié de C₁-C₃₀, éventuellement insaturé ou hydroxylé, ou un résidu d'aminoacide, de peptide ou de sucre.

5. Utilisation selon l'une quelconque des revendication 1 à 4, **caractérisée en ce que** le ou les dérivés d'oxamate sont présents en une quantité allant de 0,01 à 10 % et de préférence de 0,005 à 5 % du poids total de la composition.

6. Utilisation selon l'une quelconque des revendications 1 à 5, **caractérisée en ce que** la composition comprend en outre, au moins un actif choisi parmi les agents kératolytiques et/ou desquamants, anti-inflammatoires, apaisants, autres agents dépigmentants et leurs mélanges.

7. Procédé cosmétique de dépigmentation et/ou blanchiment de la peau humaine, des poils ou des cheveux, **caractérisé en ce qu'**il consiste à appliquer sur la peau, les poils ou les cheveux une composition comprenant au moins un dérivé d'oxamate présentant la formule (I) telle que définie dans la revendication 1.

## Patentansprüche

1. Verwendung mindestens eines Oxamatderivats der folgenden Formel (I): 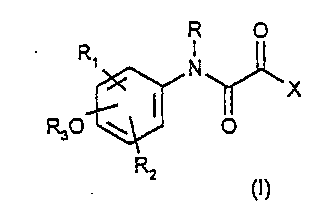 worin bedeuten:
R ein Wasserstoffatom oder eine Gruppe, die ausgewählt ist unter: Hydroxy, -OR', -COR', -COOR', -NR'R", -CONR'R", aliphatischen, gesättigten oder ungesättigten, geradkettigen, cyclischen oder verzweigten C₁₋₃₀-Kohlenwasserstoffgruppen, die gegebenenfalls hydroxyliert sind, und substituierten oder unsubstituierten Arylgruppen;
R₁ und R₂, die identisch oder voneinander verschieden sind, ein Wasserstoffatom oder eine Gruppe, die ausgewählt ist unter: Hydroxy, -OR', -COR', -COOR', -NR'R", -CONR'R", -SR', -CH₂OR', aliphatischen, gesättigten oder ungesättigten, geradkettigen, cyclischen oder verzweigten C₁₋₃₀-Kohlenwasserstoffgruppen, die gegebenenfalls hydroxyliert sind, und substituierten oder unsubstituierten Arylgruppen;
wobei R' und R", die identisch oder voneinander verschieden sind, ein Wasserstoffatom oder eine Gruppe bedeuten, die ausgewählt ist unter: den aliphatischen, gesättigten oder ungesättigten, geradkettigen oder verzweigten C₁₋₃₀-Kohlenwasserstoffgruppen, die gegebenenfalls hydroxyliert sind, substituierten oder unsubstituierten Arylgruppen, und Aminosäure- oder Zuckerresten;
R₃ ein Wasserstoffatom;
X eine Gruppe, die unter -OR₅, -SR₅ und -NR₆R₇ ausgewählt ist,
wobei R₅, R₆ und R₇, die identisch oder voneinander verschieden sind, ein Wasserstoffatom oder eine Gruppe bedeuten, die ausgewählt ist unter: den aliphatischen, gesättigten oder ungesättigten, geradkettigen, cyclischen oder verzweigten C₁₋₃₀-Kohlenwasserstoffgruppen, die gegebenenfalls hydroxyliert sind, substituierten oder unsubstituierten Arylgruppen, und,Aminosäure-, Peptid- oder Zukkerresten,
zur Herstellung einer Zusammensetzung, die dazu vorgesehen ist, die menschliche Haut zu depigmentieren und/oder zu bleichen und/oder Pigmentflecken der Haut zu entfernen und/oder die Körperhaare und/oder die Haare zu depigmentieren.

2. Kosmetische Verwendung mindestens eines Oxamatderivats der Formel (I) nach Anspruch 1 in einer kosmetischen Zusammensetzung als Inhibitor der Tyrosinase und/oder Melaninsynthese.

3. Verwendung einer kosmetischen Zusammensetzung, die mindestens ein Oxamatderivat der Formel (I) nach Anspruch 1 enthält, als topische Zusammensetzung zur Depigmentierung und/oder zum Bleichen der menschlichen Haut, der Körperhaare oder der Haare.

4. Verwendung nach einem der vorhergehenden Ansprüche, **dadurch gekennzeichnet, daß** die Oxamatderivate mindestens eine und vorzugsweise alle folgenden Bedingungen erfüllen:
- R₃ bedeutet Wasserstoff;
- die Gruppe -OR₃ befindet sich in ortho-Stellung oder vorteilhaft in para-Stellung an der Phenylgruppe;
- R bedeutet Wasserstoff oder eine geradkettige oder verzweigte, aliphatische C₁₋₃₀-Kohlenwasserstoffgruppe, die gegebenenfalls ungesättigt oder hydroxyliert ist;
- X ist eine Gruppe -OR₅, wobei R₅ vorteilhaft ein Wasserstoffatom oder eine geradkettige oder verzweigte, aliphatische C₁₋₃₀-Kohlenwasserstoffgruppe bedeutet, die gegebenenfalls ungesättigt oder hydroxyliert ist, oder X ist eine Gruppe -NR₆R₇, wobei die Gruppen R₆ und R₇, die identisch oder voneinander verschieden sind, vorteilhaft ein Wasserstoffatom, eine geradkettige oder verzweigte, aliphatische C₁₋₃₀-Kohlenwasserstoffgruppe, die gegebenenfalls ungesättigt oder hydroxyliert ist, oder einen Aminosäure-, Peptid- oder Zuckerrest bedeuten.

5. Verwendung nach einem der Ansprüche 1 bis 4, **dadurch gekennzeichnet, daß** das Oxamatderivat oder die Oxamatderivate in Mengenanteilen von 0,01 bis 10 % und vorzugsweise 0,005 bis 5 % des Gesamtgewichts der Zusammensetzung vorliegen.

6. Verwendung nach einem der Ansprüche 1 bis 5, **dadurch gekennzeichnet, daß** die Zusammensetzung ferner mindestens einen Wirkstoff enthält, der unter den Keratolytika und/oder abschuppenden Mitteln, entzündungshemmenden Mitteln, reizlindernden Mitteln und anderen depigmentierenden Mitteln und deren Gemischen ausgewählt ist.

7. Kosmetisches Verfahren, um die menschliche Haut, die Körperhaare und/oder die Haare zu depigmentieren und/oder zu bleichen, **dadurch gekennzeichnet, daß** auf die Haut, die Körperhaare und/oder die Haare eine Zusammensetzung aufgetragen wird, die mindestens ein in Anspruch 1 definiertes Oxamatderivat der Formel (I) enthält.

## Claims

1. Use or at least one oxamate derivative of formula (I) below: 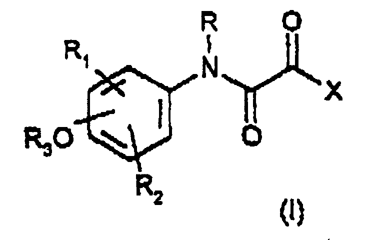 in which:
R represents a hydrogen atom, a radical chosen from a hydroxyl radical, a radical -OR', a radical -COR', a radical -COOR', a radical -NR'R", a radical -CONR'R", a linear, cyclic or branched, saturated or unsaturated, C₁ to C₃₀ aliphatic hydrocarbon radical, which is optionally hydroxylated, and a substituted or unsubstituted aryl radical,
R₁ and R₂, which may be identical or different, represent a hydrogen atom, a radical chosen from a hydroxyl radical, a radical -OR', a radical -COR', a radical -COOR', a radical -NR'R", a radical -CONR'R", a radical -SR', -CH₂OR', a linear, cyclic or branched, saturated or unsaturated, C₁ to C₃₀ aliphatic hydrocarbon radical, which is optionally hydroxylated, and a substituted or unsubstituted aryl radical,
with R' and R", which may be identical or different, representing a hydrogen atom, a radical chosen from a linear or branched, saturated or unsaturated, C₁ to C₃₀ aliphatic hydrocarbon radical, which is optionally hydroxylated, and a substituted or unsubstituted aryl radical, an amino acid residue or a sugar residue,
R₃ represents a hydrogen atom,
X represents a radical chosen from a radical -OR₅, a radical SR₅ and a radical -NR₆R₇,
with R₅, R₆ and R₇, which may be identical or different, representing a hydrogen atom, a radical chosen from a linear, cyclic or branched, saturated or unsaturated, C₁ to C₃₀ aliphatic hydrocarbon radical, which is optionally hydroxylated, and a substituted or unsubstituted aryl radical, an amino acid residue, a peptide residue or a sugar residue, for obtaining a composition intended for depigmenting and/or bleaching human skin and/or for removing skin pigmentation marks and/or for depigmenting head hair and/or other hairs.

2. Cosmetic use of at least one oxamate derivative of formula (I), as defined in Claim 1, in a cosmetic composition, as a tyrosinase inhibitor and/or as a melanin synthesis inhibitor.

3. Use of a cosmetic composition comprising at least one oxamate derivative of formula (I), as defined in Claim 1, as a topical composition for depigmenting and/or bleaching human skin, head hair or other hairs.

4. Use according to any one of the preceding claims, **characterized in that** the oxamate derivatives are chosen from those for which at least one, and preferably all, of the following conditions are satisfied:
- R₃ represents a hydrogen atom,
- the function -OR₃ on the phenyl radical is in an ortho position or, advantageously, in the para position,
- R is a hydrogen atom or a linear or branched, C₁-C₃₀ aliphatic hydrocarbon radical, which is optionally unsaturated or hydroxylated,
- X is a radical -OR₅, advantageously with R₅ representing a hydrogen atom or a linear or branched C₁-C₃₀ aliphatic hydrocarbon radical, which is optionally unsaturated or hydroxylated, or X is a radical -NR₆R₇ advantageously with R₆ and R₇, which may be identical or different, representing a hydrogen atom, a linear or branched C₁-C₃₀ aliphatic hydrocarbon radical, which is optionally unsaturated or hydroxylated, an amino acid residue, a peptide residue or a sugar residue.

5. Use according to any one of Claims 1 to 4, **characterized in that** the oxamate derivative or derivatives are present in an amount ranging from 0.01 to 10% and preferably from 0.005 to 5% of the total weight of the composition.

6. Use according to any one of Claims 1 to 5, **characterized in that** the composition also comprises at least one active agent chosen from keratolytic agents and/or desquamating agencs, anti-inflammatory agents, calmants, other depigmenting agents and mixtures thereof.

7. Cosmetic process for depigmenting and/or bleaching human skin, head hair or other hairs, **characterized in that** it consists in applying a composition, comprising at least one oxamate derivative of formula (I) as defined in Claim 1, to the skin, head hair or other hairs.
